# EUROPÄISCHE PATENTSCHRIFT

(11) **EP 1 654 023 B1**
(45) Veröffentlichungstag und Bekanntmachung des Hinweises auf die Patenterteilung: **16.07.2014**
(21) Anmeldenummer: 04762494.5
(22) Anmeldetag: 23.07.2004
(51) Int. Cl.: A61M 16/00, A61F 2/20, A61M 16/04, A61M 25/00

(54) **ANORDNUNG ZUR ATMUNGSUNTERSTÜTZUNG EINES PATIENTEN**
ARRANGEMENT FOR RESPIRATORY SUPPORT FOR A PATIENT
ENSEMBLE PERMETTANT L'ASSISTANCE RESPIRATOIRE D'UN PATIENT

(30) Priorität: 11.08.2003 DE 10337138
(43) Veröffentlichungstag der Anmeldung: 10.05.2006
(73) Patentinhaber: Breathe Technologies, Inc., Fremont, CA 94539 (US)
(72) Erfinder: Freitag, Lutz, 58675 Hemer (DE)
(74) Vertreter: Carter, Stephen John
(86) Internationale Anmeldenummer: PCT/DE2004/001646
(87) Internationale Veröffentlichungsnummer: WO 2005/014091

(56) Entgegenhaltungen:
- EP-A- 0 778 035
- EP-A- 0 778 035
- EP-A1- 0 602 734
- EP-A1- 0 692 273
- WO-A2-01/76655
- DE-A- 19 626 924
- DE-A- 19 626 924
- US-A- 4 971 049
- US-A- 5 184 610
- US-A- 5 184 610
- US-A- 5 400 778
- US-A- 5 400 778
- US-A- 5 954 050
- US-B1- 6 371 114

## Beschreibung

Die Erfindung betrifft eine Anordnung zur Atmungsunterstützung eines Patienten.

Damit der Körper Sauerstoff aufnehmen und Kohlendioxid abgeben kann, müssen beide Komponenten des respiratorischen Bronchialsystems funktionieren. Die Lunge als gasaustauschendes Organ und die Atempumpe als Ventilationsorgan, welches die Luft in die Lunge hinein und wieder hinaus transportiert. Zur korrekten Funktion der Atempumpe gehören das Atemzentrum im Gehirn, zentrale und periphere Nerven, der knöcherne Thorax und die Atemmuskulatur sowie freie, stabile Atemwege.

Bei bestimmten Erkrankungen besteht eine dauerhafte Überbeanspruchung oder Erschöpfung der Atempumpe. Ein typisches Krankheitsbild ist das Lungenemphysem mit flach stehenden Zwerchfellen ohne Kontraktionsfähigkeit. Bei einem Lungenemphysem sind die Atemwege meistens extrem schlaff und kollaptisch. In Folge der abgeflachten, überdehnten Zwerchfelle kann der Patient nicht tief genug einatmen. Zusätzlich kann der Patient aufgrund der kollabierenden Atemwege nicht ausreichend genug ausatmen. Dies führt zu einer unzureichenden Atmung mit Sauerstoffunterversorgung und einem Anstieg des Kohlendioxids im Blut, zur so genannten ventilatorischen Insuffizienz.

Die Behandlung der Einatmungsschwäche erfolgt oft über ein Beatmungsgerät. Die so genannte Heimbeatmung ist eine künstliche Beatmung zur Unterstützung oder vollständigen Entlastung der Atempumpe.

Die Beatmung kann nicht-invasiv erfolgen über einen Schlauch und eine Nasen- oder Mund/Nasenmaske, die der Patient bei Bedarf selbst auf- und absetzen kann. Hierdurch wird jedoch die freie Atmung und das Sprechen des Patienten behindert. Des Weiteren kann eine geblockte Trachealkanüle in die Luftröhre eingesetzt werden. Auch dies hat zur Folge, dass der Patient nicht mehr sprechen kann.

Bei der invasiven Beatmung geschieht dies meistens über ein Tracheostoma. Hierbei handelt es sich um eine operativ angelegte Öffnung in der Luftröhre. Über die Öffnung wird ein fingerdicker Katheter mit einem Blockungsballon in die Luftröhre eingeführt und dieser an ein Beatmungsgerät angeschlossen. Dies ermöglicht eine ausreichend tiefe Atmung, hindert den Patienten aber am Sprechen. Neben der Beatmung gibt es die transtracheale Sauerstoffgabe über dünnere Katheter. Entsprechende Vorschläge gehen aus der US-A-5,181,509 oder der US-A-5,279,288 hervor. Auf diese Weise wird dem Patienten eine hochdosierte Sauerstoffgabe in einem kontinuierlichen Strahl mit fest eingestellter Frequenz verabreicht. Eine Regulierung des Sauerstoffstroms wird manuell über eine Drosseleinrichtung vorgenommen. Eine Anpassung auf den natürlichen Atmungsvorgang eines Patienten ist nicht möglich. Die Atmung wird nicht vertieft. Auch kann das in die Luftröhre eingeführte Katheterende zu Irritationen und einer lokalen Traumatisierung des umliegenden Gewebes führen, indem es in Folge der Atembewegung gegen die Luftröhre schlägt oder das umliegende Gewebe durch den Jet-Strom ausgetrocknet wird.

Dokument US-A-5,954,050 offenbart ein System zur Atmungsunterstützung eines Patienten mit Spontanatmung, das System umfassend eine Sauerstoffpumpe zum Anschluss an eine Sauerstoffquelle, einen Luftröhrenkatheter der über sein erstes Ende mit der Sauerstoffpumpe verbunden ist, und ein zweites Ende zur transträchealen Einbringung in die Luftröhre eines Patienten aufweist, wobei der Katheter dem Patienten ermöglicht die Umgebungsluft spontan ein- und auszuatmen. Das System weist auch einen Sensor auf, der durch Einbringung in die Luftröhre des Patienten zur Erfassung der Spontanatmung des Patienten angepasst ist , und eine mit dem Sensor verbundene Steuereinheit.

Bekannt sind ferner so genannte "Montgomery-T-Röhrchen", die in die Luftröhre eingelegt werden. Hierüber kann der Patient über den nach außen geleiteten Schenkel des T-Stücks Sauerstoff erhalten. Ferner kann sich der Patient im Bedarfsfall selber Sekret absaugen. Der Patient kann frei atmen und bei Verschluss des vorderen Schenkels sprechen, eine Beatmung ist über das "Montgomery-T-Röhrchen" aber nicht möglich, da die eingeleitete Luft nach oben in den Mund- oder Rachenraum ausweicht.

Der Erfindung liegt daher ausgehend vom Stand der Technik die Aufgabe zugrunde, eine verbesserte Anordnung zur Atmungsunterstützung eines Patienten zu schaffen, die sicher in der Anwendung ist. Es werden auch eine Luftröhrenprothese und einen Katheter beschrieben, die eine mit der Spontanatmung des Patienten synchronisierte Atmungsunterstützung ermöglichen, ohne die Sprechfähigkeit zu beeinträchtigen.

Die Lösung des verfahrensmäßigen Teils der Aufgabe besteht in einer Anordnung nach dem Patentanspruch 1.

Bei einer Anwendung der Anordnung wird die Spontanatmung des Patienten sensorisch erfasst und am Ende eines Einatmungsvorgangs der Lunge eine zusätzliche Sauerstoffmenge verabreicht. Dies kann in Form eines Sauerstoffstoßes über einen Jet-Katheter aus einem Sauerstoffreservoir erfolgen. Hierbei erfolgt eine Synchronisation der Atmungsunterstützung mit der natürlichen Atmung eines Patienten. Auf diese Weise wird die infolge einer Überbeanspruchung oder Erschöpfung der Atempumpe verminderte Atemtiefe kompensiert. Die Atmung wird durch die zusätzliche Sauerstoffmenge auf einem ausreichenden Niveau gehalten. Eine Sauerstoffunterversorgung und der Anstieg von Kohlendioxid im Blut wird vermieden.

Zweckmäßigerweise hat die zusätzliche Sauerstoffmenge ein Volumen zwischen 25 ml bis 150 ml.

Wahlweise kann bei Bedarf der Ausatmungsvorgang des Patienten durch einen Gegenstrom gebremst werden. Dies ist immer dann empfehlenswert, wenn die Atemwege des Patienten kollaptisch sind, also im Augenblick des Ausatmungsvorgangs zusammenfallen, wodurch der Ausatmungsvorgang extrem behindert werden kann. Dies kann dadurch verhindert werden, dass während der Ausatmung ein Gegenstrom aufgebracht wird, wodurch die Atemwege offen gehalten und ein Kollaps der Atemwege vermieden wird.

Die erfinderische Anordnung sieht eine an eine Sauerstoffquelle anschließbare Sauerstoffpumpe und eine Luftröhrenprothese vor, die über einen Katheter gegebenenfalls unter weiterer Verwendung eines Zuführschlauchs verbindbar sind. Das ausströmseitige Ende des Katheters wirkt einen Düsencharakter auf den Sauerstoffstrom aus. Dies kann beispielsweise durch eine Querschnittsverringerung erfolgen. Grundsätzlich kann das Ende des Katheters auch mit einer Jet-Düse versehen sein. Ferner sind Sensoren vorgesehen zur Erfassung der Spontanatmung des Patienten, die mit einer Steuereinheit zur Aktivierung der Sauerstoffpumpe verknüpft sind. Die Luftröhrenprothese weist einen tubulären Stützkörper mit einem Anschluss für den Katheter auf. Der Stützkörper und der integrierte Katheter sind so dimensioniert, dass der Patient ungehindert frei atmen und sprechen kann. Die Hauptatmung erfolgt durch das größere Innenlumen der Luftröhrenprothese. Die Spontanatmung, Husten und Sprechen werden nicht behindert. Ferner sind am Stützkörper zumindest zwei der zur Anordnung gehörenden Sensoren vorgesehen.

Die Luftröhrenprothese wird in die Luftröhre eines Patienten implantiert. Über einen kleinen Luftröhrenschnitt wird der Zugang für den Katheter nach außen geschaffen. Der Katheter kann über den Anschluss mit einem Ende direkt in den Stützkörper geführt werden. Möglich ist es auch, den Katheter außen über eine Kupplung mit dem Anschluss zu verbinden.

Die Sensoren dienen zur Erfassung der Spontanatmung des Patienten. Es können verschiedenartige A temfühler, beispielsweise A temfluss-Sensoren o der Druckfühler zur Anwendung gelangen. Besonders vorteilhaft sind Thermistoren. Hierbei handelt es sich um Halbleiterbauelemente mit temperaturabhängigem Widerstand. Die Temperaturabhängigkeit der Widerstände wird zur Erfassung der Ein- bzw. Ausatmungsvorgänge genutzt da die Ausatemluft der Lunge in der Luftröhre naturgemäß wärmer ist als die Einatemluft.

In vorteilhafter Weise ist gemäß den Merkmalen von Patentanspruch 4 ein Sensor an der Innenwand des Stützkörpers festgelegt. Der andere Sensor ist dann an der Außenwand des Stützkörpers angeordnet oder im Stützkörper selbst eingebettet.

Über eine Brückenschaltung erfolgt eine Kompensation der aufgenommenen Messwertunterschiede zwischen dem innenliegenden und dem außenliegenden Sensor. Durch diese Doppelanordnung können Umwelteinflüsse, wie Temperaturschwankungen und ähnliches ausgeglichen werden.

Nach den Merkmalen von Patentanspruch 5 ist das im Stützkörper befindliche Ende des Katheters im Wesentlichen parallel zu dessen Längsachse umgelenkt und endseitig mit einer Jet-Düse versehen. Hierbei kann es sich um eine separate Düse handeln. Die Jet-Düse kann aber auch in Form einer Querschnittsverringerung am Ende des Katheters gestaltet sein. Auf diese Weise kann der über den Katheter eingeleitete Luft- bzw. Sauerstoffstrom in Richtung zur Lunge geleitet werden, und zwar mit einer laminaren Strömung. Ein Ausweichen des Sauerstoffs in den Mund- oder Rachenraum wird verhindert. Der das Katheterende bzw. das Endstück aufnehmende Stützkörper verhindert ein Austrocknen des umliegenden Gewebes. Zudem wird eine Traumatisierung der Luftröhre bzw. des Gewebes, beispielsweise in Folge von Bewegungen des Katheterendes, vermieden.

Zweckmäßigerweise ist die Sauerstoffpumpe als Kolbenpumpe ausgeführt. Insbesondere bietet sich der Einsatz eines Zylinders mit doppelt wirkendem Kolben oder verschieblicher Membran an. Eine solche Sauerstoffpumpe zeichnet sich durch ihre kompakte Bauweise aus. Zudem ist eine zuverlässige Einstellung der aufgegebenen Sauerstoffmenge möglich, und zwar sowohl für die Unterstützung des Einatmungsvorgangs als auch die des Ausatmungsvorgangs. Da die maximale Luftmenge pro Jet-Hub durch die Zylindergöße begrenzt ist, kann auch ein Überblähen der Lunge mit der Folge eines Baro-Traumas vermieden werden.

Im Rahmen der erfindungsgemäßen Anordnung können zwei Katheter zur Anwendung gelangen, wobei ein Jet-Katheter für die Unterstützung des Einatmungsvorgangs und der andere Katheter für das gezielte Abbremsen des Ausatmungsvorgangs vorgesehen ist. Selbstverständlich kann auch ein Katheter doppellumig ausgeführt sein, wie dies Patentanspruch 7 vorsieht. Über den doppellumigen Katheter werden getrennte Kanäle für die Verabreichung von Sauerstoff beim Einatmungsvorgang und beim Ausatmungsvorgang bereitgestellt.

Eine Sicherheitssteigerung der Anordnung wird erzielt, wenn weitere Atemfühler vorgesehen sind. Auch hierbei handelt es sich um Sensoren zur Erfassung der Spontanatmung eines Patienten. Diese können beispielsweise am Brustkorb des Patienten befestigt werden, so dass die Spontanatmung durch eine Thoraximpedanz-Messung überwacht werden kann. Denkbar ist auch eine Schall- oder Flussmessung an Mund oder Nase des Patienten. Durch Abgleich der aufgenommenen Signale aus der Luftröhre und der weiteren Atemfühler in einer Steuer- und Kontrolleinheit und entsprechende Ansteuerung der Sauerstoffpumpe wird die Einatmungs- bzw. Ausatmungsunterstützung durchgeführt. Die zusätzlichen Atemfühler gewährleisten eine redundante Ausführung und tragen zur Sicherheit der Anordnung bei.

Eine zur Anwendung mit der Anordnung nach Anspruch 1 geeignete Luftröhrenprothese weist einen tubulären Stützkörper mit einem Anschluss für einen Katheter auf, wobei am Stützkörper zumindest zwei Sensoren angeordnet sind. Die Luftröhrenprothese zeichnet sich dadurch aus, dass mit ihr die Atmung eines Patienten messbar ist. Auf diese Weise ist eine Synchronisation der externen Atmungsunterstützung mit der Eigenatmung des Patienten möglich.

In vorteilhafter Weise i st ein Sensor an der Innenwand des Stützkörpers befestigt. Als besonders geeignet werden im Rahmen der Erfindung. Thermistoren angesehen. Durch Zusammenfassung der Thermistoren in einer Brückenschaltung kann eine Kompensation der Temperatur zwischen dem innen liegenden und einem außen liegenden Thermistor erfolgen. Diese Doppelanordnung der Sensoren in der Brückenschaltung kompensiert Umwelteinflüsse, wie Temperaturschwankungen oder auch solche, die durch Sekret hervorgerufen werden, welches sich am inneren Sensor auflegt und eine lokale Abkühlung oder Erwärmung bedingt.

Vorteilhaft ist ferner, wenn das Katheterende im Stützkörper parallel zu dessen Längsachse geführt ist. Hierdurch erfolgt eine gerichtete Aufgabe der Sauerstoffströme in Richtung zum Bronchialtrakt, und zwar mit laminaren Strömungsverhältnissen.

An dem ausströmseitigen Ende des Katheters ist zumindest ein Sensor befestigt. Zweckmäßigerweise sind dort zwei Sensoren vorgesehen, um innerhalb einer Brückenschaltung eine Kompensation von Messwertunterschieden durchführen zu können.

Ein solcher Katheter kann von außen in einen Stützkörper eingeführt werden. B ei einem solchen Stützkörper kann es sich beispielsweise um einen bekannten "Montgomery-T-Stent" handeln. Über den von außen zugängigen Schenkel des T-Stücks wird der Katheter eingeführt, um darüber die Atmung zu unterstützen.

Nach den Merkmalen von Patentanspruch 10 weist das Ende des Katheters eine Jet-Düse auf. Diese ist, wie bereits weiter oben beschrieben, beispielsweise durch eine Querschnittsverengung des Endes ausgeführt. Es kann sich aber auch um eine separate Jet-Düse handeln.

Vorteilhafterweise ist das Ende des Katheters gekrümmt ausgeführt. Auf diese Weise ist das in die Luftröhre bzw. den Stützkörper eingeführte Ende selbsttätig in Richtung zum Bronchialtrakt parallel zur Längsachse des Stützkörpers ausgerichtet.

Die Erfindung ist nachfolgend anhand der beigefügten Zeichnungen näher beschrieben. Es zeigen:
- Figur 1: den Oberkörper eines Patienten, der eine erfindungsgemäße Anordnung zur Atmungsunterstützung trägt;
- Figur 2: ein Diagramm mit der Darstellung des Atemflusses eines Emphysem-Patienten mit und ohne Atmungsunterstützung;
- Figur 3: eine technisch vereinfachte Darstellung einer Luftröhrenprothese;
- Figur 4: ein weiteres Beispiel einer Luftröhrenprothese;
- Figur 5: ebenfalls im Schema eine zur erfindungsgemäßen Anordnung gehörende Sauerstoffpumpe mit der Darstellung der Luftführung und einer Steuereinheit;
- Figur 6: den endseitigen Ausschnitt aus einem Katheter und
- Figur 7: den Katheter gemäß der Figur 6 in einem Stützkörper eingesetzt.

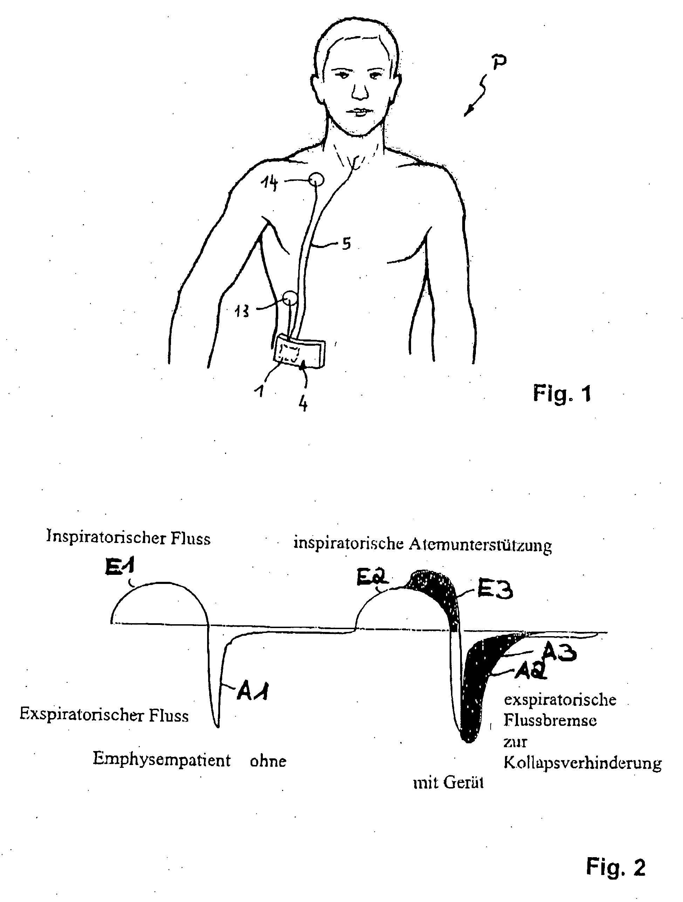

In der Figur 1 ist mit P ein Patient bezeichnet, der an einem Lungenemphysem leidet mit einer Überanspruchung und Erschöpfung der Atempumpe. Hierdurch kann der Patient nicht tief genug einatmen. Zusätzlich wird der Ausatmungsvorgang durch schlaffe und kollabierende Atemwege behindert.

Ein solcher Atmungsvorgang mit Einatmung (Inspiratorischer Fluss) und Ausatmung (Exspiratorischer Fluss) ohne Atmungsunterstützung ist in der Figur 2 in der linken Bildhälfte dargestellt. Der Kurve der Einatmung ist mit E1, der Kurve der Ausatmung mit A1 bezeichnet.

Zur Unterstützung bzw. Entlastung der Atempumpe wird bei dem Patienten die Spontanatmung sensorisch erfasst und am Ende eines Einatmungsvorgangs der Lunge eine zusätzliche Sauerstoffmenge verabreicht. Dieser Atemfluss ist in der Figur 2 in der rechten Bildhälfte verdeutlicht. Die zusätzliche Sauerstoffmenge vergrößert das Aternvolumen bei der Einatmung gemäß Kurve E2 um das in der oberen Kurve dunkel angelegte und mit E3 bezeichnete Differenzvolumen. Die zusätzliche Sauerstoffmenge kann ein Volumen zwischen 25 ml und 150 ml haben.

Zusätzlich wird der Ausatmungsvorgang des Patienten durch einen Gegenstrom gebremst. Hierdurch verlagert sich der Atemfluss bei der Ausatmung entsprechend der mit A2 bezeichneten Kurve. Durch diesen gezielten der Ausatmung entgegengesetzt wirkenden Widerstand wird ein Kollabieren der Atemwege beim Ausatmen verhindert. Auf diese Weise wird das Ausatemvolumen um das ebenfalls dunkel angelegte und mit A3 bezeichnete Volumen vergrößert.

In der Konsequenz wird durch diese Vorgehensweise eine unzureichende Atmung mit Sauerstoffunterversorgung und ein Anstieg von Kohlendioxid im Blut vermieden. Der Patient P ist wesentlich belastbarer und mobiler, zudem verspürt er weniger oder keine Luftnot.

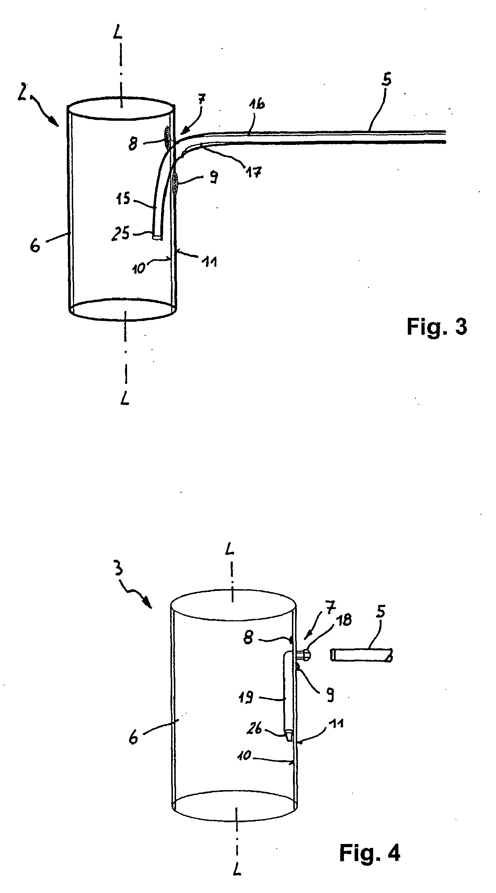

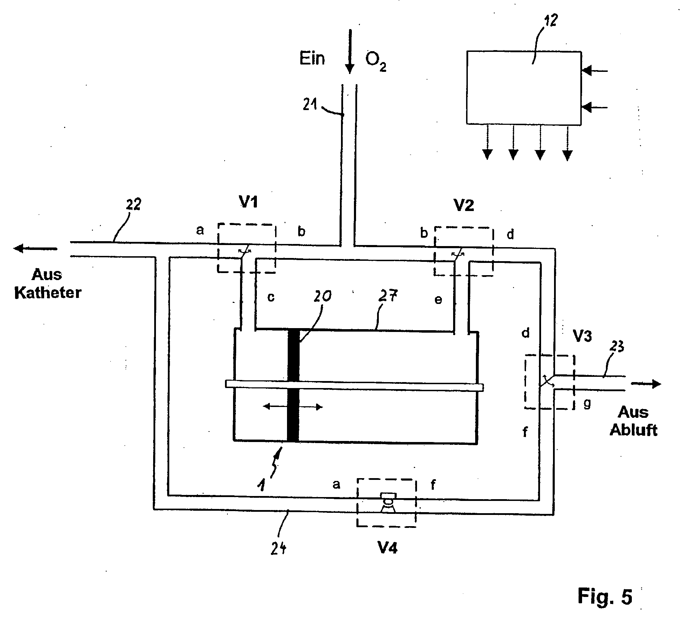

Zur Durchführung der Atmungsunterstützung des Patienten P ist eine Anordnung vorgesehen, welche eine an eine Sauerstoffquelle anschließbare Sauerstoffpumpe 1 (siehe Figur 5) und eine Luftröhrenprothese 2, 3 (siehe Figuren 3 und 4) umfasst. Gemäß Figur 1 ist die Sauerstoffpumpe 1 Bestandteil eines kompakten mobilen Beatmungsgerätes 4. Die Sauerstoffpumpe 1 und die Luftröhrenprothese 2 bzw. 3 sind über einen Katheter 5 verbunden.

Wie die Figuren 3 und 4 zeigen, weist jede Luftröhrenprothese 2 bzw. 3 einen tubulären Stützkörper 6 mit einem Anschluss 7 für den Katheter 5 auf. Zur Erfassung der Spontanatmung des Patienten P sind dem Stützkörper 6 zwei Sensoren 8,9 in Form von Thermistoren zugeordnet. Hierbei ist ein Sensor 8 an der Innenwand 10 des Stützkörpers 6 festgelegt, wohingegen sich der andere Sensor 9 an der Außenwand 11 des Stützkörpers 6 befindet. Die Sensoren 8, 9 stehen mit einer Steuereinheit 12 zur Aktivierung der Sauerstoffpumpe 2 in Verbindung. Die Steuereinheit 12 ist in der Figur 5 schematisch mit ihren Ein- und Ausgängen dargestellt. Wie bereits erwähnt, handelt es sich bei den Sensoren 8, 9 um Thermistoren, also temperaturabhängige Widerstände. Diese sind innerhalb der Anordnung in einer Brückenschaltung zusammengeschlossen, so dass eine Kompensation von Messwertunterschieden zwischen dem inneren Sensor 8 und dem äußeren Sensor 9 in Folge von Umwelteinflüssen erfolgt.

Ferner erkennt man in der Figur 1, dass weitere Atemfühler 13, 14 vorgesehen sind. Auch hierbei handelt es sich um Sensoren zur Erfassung der Spontanatmung des Patienten P. Durch Abgleich der über die Sensoren 8 und 9 bzw. 13, 14 aufgenommenen Messwerte kann man ein exaktes Bild vom Atmungsvorgang des Patienten P erhalten. Zudem wird die Sicherheit gegenüber Falschmessungen oder Ausfall eines der Sensoren 8, 9 bzw. 13, 14 erhöht.

Bei der Luftröhrenprothese 2 gemäß Figur 3 kann der Jet-Katheter 5 über den Anschluss 7 in den Stützkörper 6 eingeschoben werden. Das im Stützkörper 6 befindliche Ende 15 des Jet-Katheters 5 wird hierbei etwa parallel zu dessen Längsachse L geführt bzw. umgelenkt. Die Datenleitungen von den Sensoren 8, 9 zur Steuereinheit 12 sind mit 16 und 17 bezeichnet. Diese verlaufen innerhalb des Katheters 5. Ausströmseitig ist das ende 15 des Jet-Katheters 5 als Jet-Düse 25 gestaltet. Dies kann durch Verringerung des Katheter-Querschnitts erfolgen. Hierdurch wird die Geschwindigkeit des Sauerstoffstroms am Austritt aus dem Katheter 5 erhöht und in Richtung zum Bronchialtrakt geleitet. Der Durchmesser des Stützkörpers 6 ist so dimensioniert mit genügend freiem Lumen, dass der Patient P auch bei integriertem Katheter 5 frei atmen und sprechen kann.

Bei der Luftröhrenprothese 3 gemäß Figur 4 ist eine separate Kupplung 18 am Anschluss 7 vorgesehen, über den der Katheter 5 mit der Luftröhrenprothese 3 verbunden wird. In diesem Fall ist im Stützkörper 6 ein parallel zur Längsachse L ausgerichteter festgelegter Längenabschnitt 19 als Katheterende vorgesehen, wobei der Sauerstoffstrom über eine Jet-Düse 26 in Richtung zum Bronchialtrakt geleitet wird.

Die Sauerstoffpumpe 1 ist in der Figur 5 schematisch dargestellt. Es handelt sich um eine Kolbenpumpe mit einem in einen Zylinder 27 angeordneten doppelt wirkenden Kolben 20. Insgesamt weist die Anordnung vier Ventile V1 bis V4 auf. Die Sauerstoffzuführung erfolgt aus einem externen Sauerstoffreservoir über den Anschluss 21. Die Schaltzustände der Ventile V1 bis V4 bzw. die Zu- und Ableitungen sind durch Buchstaben a bis g gekennzeichnet.

Bei der Atmungsunterstützung ist die Funktion der Sauerstoffpumpe 1 innerhalb der Anordnung wie folgt:
Wenn das Ventil V1 von c nach a offen (b nach c geschlossen) und das Ventil V2 von b nach e offen (e nach d geschlossen) ist, bewegt sich der Kolben 20 in Bildebene nach links und der Sauerstoff strömt über den Auslass 22 und den Jet-Katheter 5 zum Patienten P. Es erfolgt die Verabreichung der zusätzlichen Sauerstoffmenge E3 beim Einatmungsvorgang des Patienten P.
Wenn das Ventil V1 von b nach c (c nach a geschlossen) offen und das Ventil V2 von e nach d offen (b nach e geschlossen) ist, dann bewegt sich der Kolben 20 in Bildebene nach rechts und die Strömung des Sauerstoffs erfolgt in Richtung zum Ventil V3. Das Ventil V3 steht über einen Auslass 23 mit der Umluft in Verbindung. Im Fall, dass das Ventil V3 von d nach g offen ist, strömt der Sauerstoff ab ohne Exspirationsbremse. Das bedeutet, der Ausatmungsvorgang wird nicht durch einen Gegenstrom gebremst.

Ist das Ventil V3 von d nach g geschlossen und von d nach f offen, strömt der Sauerstoff in Richtung über den Zuweg 24 zum Auslass 22 und den Katheter 5, um dem Patienten P beim Ausatmungsvorgang verabreicht zu werden und den Atemfluss zu bremsen. Durch den Gegenstrom wird ein Kollabieren der Atemwege verhindert und diese offen gehalten. Dies ermöglicht eine tiefere Ausatmung.

Im Zuweg 24 der Anordnung ist ferner das Ventil V4 geschaltet, über welches der Durchfluss (f nach a) variabel einstellbar ist. Dies kann vorteilhafterweise ein Proportionalventil mit Pulsbreitenmodulation sein.

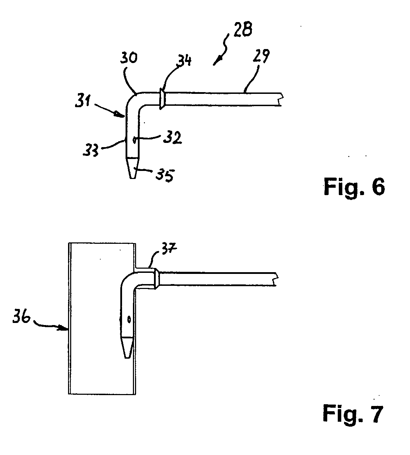

Die Figur 6 zeigt einen Katheter 28 mit einem langgestreckten, flexiblen Schlauch 29 und einem über eine Krümmung 30 abgewinkelten ausströmseitigen Ende 31. Am Ende sind zwei Sensoren 32, 33 zur Erfassung der Spontanatmung eines Patienten P befestigt. Bei den Sensoren 32, 33 handelt es sich bevorzugt um Thermistoren. Auf die Darstellung von Datenleitungen ist der Einfachheit halber verzichtet worden. Diese verlaufen durch den Katheter 28 bzw. die Katheterwand. Mit 34 ist ein Anschlag bezeichnet.

Ferner erkennt man, dass das Ende 31 des Katheters 28 mit einer Jet-Düse 35 versehen ist. In der Jet-Düse 35 ist der Strömungsquerschnitt gegenüber dem Querschnitt des Katheters verringert, so dass der zugeführte Sauerstoff in der Austrittsgeschwindigkeit erhöht wird.

Der Katheter 28 kann in einen Stützkörper 36, wie in Figur 7 dargestellt, eingeführt werden. Der Stützkörper 35 befindet sich in der Luftröhre eines Patienten P. Die Verbindung nach außen wird über einen Anschluss 37 hergestellt

Bei dem Stützkörper 36 kann es sich um einen herkömmlichen "Montgomery-T-Stent" handeln.

### Bezugszeichenaufstellung

- 1 -: Sauerstoffpumpe
- 2 -: Luftröhrenprothese
- 3 -: Luftröhrenprothese
- 4 -: Beatmungsgerät
- 5 -: -Katheter
- 6 -: Stützkörper
- 7 -: Anschluss
- 8 -: Sensor
- 9 -: Sensor
- 10 -: Innenwand v. 6
- 11 -: Außenwand v. 6
- 12 -: Steuereinheit
- 13 -: Atemfühler
- 14 -: Atemfühler
- 15 -: Ende v. 5
- 16 -: Datenleitung
- 17 -: Datenleitung
- 18 -: Kupplung
- 19 -: Längenabschnitt
- 20 -: Kolben
- 21 -: Anschluss
- 22 -: Auslass
- 23 -: Auslass
- 24 -: Zuweg
- 25 -: Jet-Düse
- 26 -: Jet-Düse
- 27 -: Zylinder
- 28 -: Katheter
- 29 -: Schlauch
- 30 -: Krümmung
- 31 -: Ende v. 28
- 32 -: Sensor
- 33 -: Sensor

- 34 -: Anschlag
- 35 -: Jet-Düse
- 36 -: Stützkörper
- 37 -: Anschluss

- P -: Patient
- E1 -: Einatmungskurve
- E2 -: Einatmungskurve
- E3 -: Volumen
- A1 -: Ausatmungskurve
- A2 -: Ausatmungskurve
- A3 -: Volumen

- V1 -: Ventil
- V2 -: Ventil
- V3 -: Ventil
- V4 -: Ventil

- L -: Längsachse v. 5

- a -: Leitung
- b -: Leitung
- c -: Leitung
- d -: Leitung
- e -: Leitung
- f -: Leitung
- g -: Leitung

## Patentansprüche

1. Anordnung zur Atmungsunterstützung eines Patienten mit Spontanatmung, wobei die Anordnung folgendes umfasst:
a) eine Sauerstoffpumpe (1) zum Anschluss an eine Sauerstoffquelle;
b) ein Luftröhrenkatheter (5, 28), der ein erstes Ende aufweist, das mit der Sauerstoffpumpe (1) verbunden ist, und ein zweites Ende zur transtrachealen Einbringung in die Luftröhre eines Patienten, derart dimensioniert, dass in der Verwendung die Spontanatmung und das Sprechen nicht behindert werden;
c) mindestens einen Sensor, (8, 9, 32, 33) zur Einbringung in die Luftröhre des Patienten zur Erfassung der Spontanatmung des Patienten; und
d) eine mit dem mindestens einen Sensor verbundene Steuereinheit (12), zur Aktivierung der Sauerstoffpumpe (1) zur Förderung einer zusätzlichen Sauerstoffmenge zum Patienten,
**dadurch gekennzeichnet dass**
die mit mindestens einem Sensor verbundene Steuereinheit konfiguriert ist, um die zusätzliche Sauerstoffmenge synchron mit der Spontanatmung des Patienten zu liefern.

2. Anordnung nach Patentanspruch 1, welche außerdem eine Luftröhrenprothese (2, 3), mit einem tubulären Stützkörper (6) umfasst, wobei der Stützkörper (6) einen Anschluss (7) für den Katheter aufweist.

3. Anordnung nach Patentanspruch 2, wobei der mindestens ein Sensor (8, 9) am Stützkörper (6) vorgesehen ist.

4. Anordnung nach Patentanspruch 3, wobei einer der mindestens einen Sensoren (8) gegen die innere Wand (10) des Stützkörpes (6) festgelegt wird.

5. Anordnung nach einem der Patentansprüche 2 bis 4, bei welcher das zweite Ende (15) des Katheters (5) sich innerhalb des Stützkörpers (6) befindet und so ausgerichtet ist, dass er sich etwa parallel zu der Längsachse (L) des Stützkörpers (6) befindet und endseitig mit einer Jet-Düse versehen ist.

6. Anordnung nach einem der Patentansprüche 1 bis 5, bei welcher die Sauerstoffpumpe (1) eine Kolbenpumpe ist.

7. Anordnung nach einem der Patentansprüche 1 bis 6, bei welcher der Katheter doppellumig ausgeführt ist.

8. Anordnung nach einem der Patentansprüche 1 bis 7, wobei einer der Sensoren (32, 33) am zweiten Ende des Katheters angeordnet ist.

9. Anordnung nach einem der Patentansprüche 1 bis 8, bei welchem weitere Atemfühler (13, 14) zusätzlich zu dem mindestens einen Sensor (8, 9, 32, 33) vorgesehen sind.

10. Anordnung nach einem der Patentansprüche 1 bis 9, bei welcher der Katheter (5) eine Jet-Düse hat und der Querschnitt der Jet-Düse kleiner als der Querschnitt des Katheters ist, so dass der Volumenstrom des zugeführten Sauerstoffs erhöht wird.

11. Anordnung nach jedem der Patentansprüche 1 bis 10, bei welcher der Sensor aus einem Paar Thermistoren besteht.

12. Anordnung nach Anspruch 11, bei welcher die Thermistoren zum Ausgleich von Umwelteinflüssen in einer Brückenschaltung verbunden sind.

13. Anordnung nach Patentanspruch 1, bei welcher besagter mindestens eine Sensor zwei Sensoren (8, 9) umfasst.

## Claims

1. Arrangement for respiratory support for a patient with spontaneous breathing, the arrangement comprising the following:
a) an oxygen pump (1) for connection to an oxygen source;
b) a tracheal catheter (5, 28) having a first end which is connected to the oxygen pump (1) and a second end for transtracheal insertion into the trachea of a patient, which is dimensioned such that spontaneous breathing and speech are not impeded in use;
c) at least one sensor (8, 9, 32, 33) for insertion in the trachea of the patient for detecting the spontaneous breathing of the patient, and
d) a control unit (12), connected to the at least one sensor, for activating the oxygen pump (1) in order to deliver an additional quantity of oxygen to the patient,
**characterised in that**
the control unit connected to at least one sensor is configured to deliver the additional oxygen synchronously with the spontaneous breathing of the patient.

2. Arrangement according to Claim 1, which also includes a tracheal prosthesis (2, 3) having a tubular support body (6), wherein the support body (6) has a connection (7) for the catheter.

3. Arrangement according to Claim 2, wherein the at least one sensor (8, 9) is provided on the support body (6).

4. Arrangement according to Claim 3, wherein one of the at least one sensors (8) is fastened against the inner wall (10) of the support body (6).

5. Arrangement according to any one of Claims 2 to 4, wherein the second end (15) of the catheter (5) is located inside the support body (6) and is oriented in such a way that it is located approximately parallel to the longitudinal axis (L) of the support body (6) and is provided at its end with a jet nozzle.

6. Arrangement according to any one of Claims 1 to 5, wherein the oxygen pump (1) is a piston pump.

7. Arrangement according to any one of Claims 1 to 6, wherein the catheter has a double lumen.

8. Arrangement according to any one of Claims 1 to 7, wherein one of the sensors (32, 33) is arranged at the second end of the catheter.

9. Arrangement according to any one of Claims 1 to 8, wherein further breathing sensors (13, 14) are provided additionally to the at least one sensor (8, 9, 32, 33).

10. Arrangement according to any one of Claims 1 to 9, wherein the catheter (5) has a jet nozzle and the cross section of the jet nozzle is smaller than the cross section of the catheter, so that the volume flow rate of the oxygen delivered is increased.

11. Arrangement according to each of claims 1 to 10, wherein the sensor consists of a pair of thermistors.

12. Arrangement according to Claim 11, wherein the thermistors are connected in a bridge circuit in order to compensate for environmental influences.

13. Arrangement according to Claim 1, wherein said at least one sensor comprises two sensors (8, 9).

## Revendications

1. Ensemble permettant l'assistance respiratoire d'un patient ayant une respiration spontanée, l'ensemble comprenant ce qui suit :
a) une pompe à oxygène (1) destinée à être raccordée à une source d'oxygène ;
b) un cathéter de trachée (5, 28) qui présente une première extrémité qui est reliée avec la pompe à oxygène (1) et une deuxième extrémité destinée à l'installation transtrachéale dans la trachée d'un patient, dimensionnée de telle sorte que l'utilisation n'entrave pas la respiration spontanée et la parole ;
c) au moins un capteur (8, 9, 32, 33) destiné à être installé dans la trachée du patient afin de détecter la respiration spontanée du patient ; et
d) une unité de commande (12) reliée avec l'au moins un capteur destinée à activer la pompe à oxygène (1) afin d'amener une quantité supplémentaire d'oxygène au patient, **caractérisé en ce que**
l'unité de commande reliée avec l'au moins un capteur est conçue pour fournir la quantité supplémentaire d'oxygène de façon synchronisée avec la respiration spontanée du patient.

2. Ensemble selon la revendication 1, qui comprend en outre une prothèse de trachée (2, 3) avec un élément d'appui tubulaire (6), étant entendu que l'élément d'appui (6) présente un raccord (7) pour le cathéter.

3. Ensemble selon la revendication 2, dans lequel l'au moins un capteur (8, 9) est prévu sur l'élément d'appui (6).

4. Ensemble selon la revendication 3, dans lequel l'un des au moins un capteur (8) est fixé contre la paroi intérieure (10) de l'élément d'appui (6).

5. Ensemble selon l'une des revendications 2 à 4, dans lequel la deuxième extrémité (15) du cathéter (5) se trouve à l'intérieur de l'élément d'appui (6), elle est orientée de telle sorte qu'elle est essentiellement parallèle à l'axe longitudinal (L) de l'élément d'appui (6) et elle est pourvue à son extrémité d'une buse d'injection.

6. Ensemble selon l'une des revendications 1 à 5, dans lequel la pompe à oxygène (1) est une pompe à piston.

7. Ensemble selon l'une des revendications 1 à 6, dans lequel le cathéter est réalisé de façon cloisonnée.

8. Ensemble selon l'une des revendications 1 à 7, dans lequel l'un des capteurs (32, 33) est agencé à la deuxième extrémité du cathéter.

9. Ensemble selon l'une des revendications 1 à 8, dans lequel d'autres détecteurs de respiration (13, 14) sont prévus en complément à l'au moins un capteur (8, 9, 32, 33).

10. Ensemble selon l'une des revendications 1 à 9, dans lequel le cathéter (5) possède une buse d'injection et la section de la buse d'injection est plus petite que la section du cathéter, de telle sorte que le débit volume de l'oxygène amené est augmenté.

11. Ensemble selon l'une des revendications 1 à 10, dans lequel le capteur est constitué d'une paire de thermistors.

12. Ensemble selon la revendication 11, dans lequel les thermistors sont reliés dans un montage en pont afin de compenser les facteurs ambiants.

13. Ensemble selon la revendication 1, dans lequel ledit au moins un capteur comprend deux capteurs (8, 9).
